# DEMANDE DE BREVET EUROPEEN

(11) **EP 3 459 507 A2**
(43) Date de publication de la demande: **27.03.2019**
(21) Numéro de dépôt: 18191605.7
(22) Date de dépôt: 30.08.2018
(51) Int. Cl.: A61F 5/14, A43B 7/14, A43B 17/00, A43B 7/28, A43D 1/02

(54) **PROCÉDÉ DE FORMAGE DE SEMELLE DANS UNE EMPREINTE NÉGATIVE, ET DISPOSITIF ET SYSTÈME DE FORMAGE DE SEMELLE POUR SA MISE EN OEUVRE**

(30) Priorité: 30.08.2017 FR 1770902
(71) Demandeur: Capron Podologie, 71210 Ecuisses (FR)
(72) Inventeur: FAVRE-FELIX, François-Xavier, 71640 GIVRY (FR); CECCHINI, Arnaud, 71100 CHALON-SUR-SAONE (FR); AUCLAIR, Yannick, 71190 LA CHAPELLE SOUS UCHON (FR)
(74) Mandataire: Cabinet Chaillot

(57) **Abrégé**

L'invention concerne un procédé de formage d'une semelle dans une empreinte négative réalisée sur une poche d'empreinteur, comprenant une étape de positionnement d'une paroi en matière étanche, souple et extensible, en regard de la semelle placée dans l'empreinte, de façon à former entre eux un espace de formage, et une étape de formage sous vide, comprenant faire le vide dans l'espace de formage tout en assurant une étanchéité permettant l'établissement du vide, ce par quoi la paroi d'appui vient appuyer la semelle contre l'empreinte négative pour lui donner la forme de cette dernière, et maintenir le vide pendant une durée suffisante pour que la semelle soit conformée.

L'invention concerne également un dispositif et un système de formage pour la mise en oeuvre de ce procédé.

## Description

La présente invention concerne le domaine de la fabrication de semelles sur mesure, notamment de semelles orthopédiques, et en porte en particulier sur un procédé de formage d'une semelle dans une empreinte négative de pied, sur un dispositif de formage de semelle conçu pour la mise en oeuvre de ce procédé, et sur un système de formage de semelle comprenant au moins un tel dispositif de formage de semelle.

Une semelle orthopédique, de confort, ou orthèse plantaire, est fabriquée sur mesure par un praticien de façon à être adaptée au pied du patient. Ainsi, il est essentiel que la semelle, une fois conformée, soit la plus fidèle possible à l'empreinte du pied qui a été réalisée.

Actuellement, cette conformation est obtenue par thermoformage de la semelle, qui est donc une semelle thermoformable, sur une empreinte positive ou dans une empreinte négative.

Le thermoformage sur une empreinte positive suppose, avant l'étape de thermoformage en elle-même, la réalisation d'un positif en matière rigide reproduisant initialement le pied et qui pourra par la suite subir des transformations de façon à définir une empreinte positive ayant la forme que l'on souhaite donner à la semelle.

Une solution pour thermoformer une semelle dans une empreinte négative a été développée afin d'éviter ces opérations supplémentaires fastidieuses avant l'étape de thermoformage. Cette solution consiste en un système de réalisation sous vide d'une empreinte négative, appelé couramment « empreinteur ».

Un empreinteur comprend de manière classique un ensemble poche d'empreinteur et une source de vide reliée à l'ensemble poche d'empreinteur de façon à pouvoir créer un vide dans l'ensemble poche d'empreinteur.

L'ensemble poche d'empreinteur comprend d'une manière générale une poche d'empreinteur rigidifiable par le vide et des moyens de raccordement servant à relier l'intérieur de la poche d'empreinteur à ladite source de vide. La poche d'empreinteur peut se présenter sous la forme d'une vessie en matière souple, étanche et extensible, dont les parois latérales et le fond sont éventuellement rigidifiées, ou d'une enceinte dont les parois latérales et le fond sont rigides. Dans tous les cas, la paroi formant la face supérieure est en matière souple, étanche et extensible, et la poche d'empreinteur est remplie d'une charge, notamment des microbilles ou des micro-granules, qui autorise une déformation de la face supérieure de la poche d'empreinteur.

Ainsi, en pratique, le patient se tient debout et pose son pied sur la face supérieure de la poche d'empreinteur, créant dans celle-ci un renfoncement épousant la forme du pied. Le praticien peut ensuite modeler la poche d'empreinteur, en modifiant ce renfoncement pour lui donner la forme souhaitée et obtenir ainsi l'empreinte négative devant servir au thermoformage de la semelle. Une fois que cette empreinte négative est obtenue, la source de vide est commandée de façon à créer un vide dans la poche d'empreinteur, ce qui rigidifie cette dernière et fige l'empreinte négative.

Ensuite, une semelle thermoformable, qui aura été chauffée au préalable à une température par exemple comprise entre 70°C et 110°C, est mise en place dans l'empreinte négative, le patient pose son pied sur la semelle, dans l'empreinte négative, et se tient debout de façon à exercer sur la semelle une pression et ainsi l'appuyer contre l'empreinte négative par son propre poids, lui donnant la forme de cette dernière. Le patient doit habituellement rester dans cette position pour une durée allant de 3 à 15 minutes, le temps que la semelle refroidisse jusqu'à une température où elle est rigide. Une fois ce délai écoulé, le patient peut enlever son pied de la poche d'empreinteur et la semelle conformée peut être retirée de l'empreinte négative.

Ce procédé de formage de semelle permet d'obtenir dans la plupart des cas une semelle correctement conformée.

Ce procédé de formage de semelle présente cependant l'inconvénient d'obliger le patient à rester debout avec le ou les pieds sur la ou les semelles. Cette position debout, statique, pose par son inconfort des difficultés à certains patients qui ont des difficultés à rester debout avec un pied sur la semelle pendant un temps suffisant pour le refroidissement de la semelle, de sorte que la semelle ne sera pas correctement conformée. De plus, il existe un risque que l'appui sur la semelle ne soit pas correct ou soit imprécis, de sorte que la forme de la semelle conformée ne correspondra pas parfaitement à celle de l'empreinte négative. Ce risque est amplifié pour les patients présentant des difficultés à rester debout sur la poche d'empreinteur ou risquant de bouger pendant la phase de formage de la semelle. Par ailleurs, il existe encore des réticences chez certains praticiens à utiliser ce procédé car il requiert que le pied du patient vienne en contact avec une semelle encore chaude.

La présente invention vise ainsi à proposer un procédé de formage d'une semelle dans une empreinte négative dans lequel le patient n'intervient pas dans l'étape de formage à proprement dite, ainsi qu'un dispositif pour mettre en oeuvre ce procédé.

Le brevet allemand DE 12 34 355 B divulgue un procédé de formage de semelle selon le préambule de la revendication 1 et un dispositif de formage de semelle selon le préambule de la revendication 3.

En particulier, un dispositif selon ce brevet comprend une paroi étanche, souple et extensible, destinée à être posée en regard de la face d'une poche d'empreinteur dans lequel est réalisée une empreinte négative, constituant ainsi un espace de formage dans lequel une pression négative sera établie lors de l'opération de formage proprement dit. La poche d'empreinteur se présente sous la forme d'un bac dont le fond et l'enveloppe latérale sont rigides, bac en travers duquel s'étend ladite face, réalisée en caoutchouc, formant au-dessous d'elle un espace divisé en deux chambres par une paroi perméable à l'air. La chambre supérieure, sous la face et au-dessus de la paroi, est remplie d'une charge, ici des billes, tandis que la chambre inférieure héberge un dispositif de génération de pression négative commandé par un interrupteur au pied.

L'empreinte négative est réalisée de manière classique par établissement, après que le patient a placé son pied sur la poche d'empreinteur, d'une pression négative dans ladite chambre supérieure de façon à figer la poche d'empreinteur.

Dans l'étape de formage à proprement dit, une pression négative est établie de nouveau dans ladite chambre supérieure, et également dans ledit espace de formage, à l'aide du dispositif de génération de pression négative, l'air initialement présent dans ledit espace de formage étant aspiré vers ladite chambre supérieure en traversant la face et l'air présent dans ladite chambre supérieure étant aspiré en passant par ladite paroi perméable à l'air.

On comprend donc que la poche d'empreinteur sera déformée lors de l'établissement du vide dans l'espace de formage, ce qui conduira à la perte de la forme souhaitée par le praticien : la semelle serait correctement conformée à l'empreinte négative, mais cette dernière ne correspondra plus à l'empreinte souhaitée par le praticien.

La solution selon la présente invention repose sur l'utilisation, à la place du pied du patient, d'une paroi en matière étanche, souple et extensible, que l'on place en regard de l'empreinte négative après que la semelle y a été mise en place, et sur l'établissement d'un vide seulement entre ladite paroi et l'empreinte négative de sorte que ladite paroi sera plaquée contre la semelle et l'empreinte négative et exercera une pression uniforme sur toute la semelle, sans que l'empreinte négative ne soit déformée par l'établissement du vide. La semelle est ainsi parfaitement conformée sans faire appel au patient. De plus, le refroidissement de la semelle sera accéléré du fait de l'absence du pied du patient, ce qui permet de réduire le temps nécessaire pour le formage de la semelle. A noter que la surface supérieure de la semelle en cours de formage est désormais libérée, autorisant également d'accélérer encore le refroidissement par l'ajout d'un refroidisseur.

La présente invention a donc pour objet un procédé de formage d'une semelle dans une empreinte négative, comprenant les étapes successives suivantes :
- une étape de mise en place d'une semelle dans une empreinte négative réalisée sur une face d'une poche d'empreinteur, ladite face étant en matière souple, extensible, et la poche d'empreinteur étant remplie d'une charge qui autorise une déformation de ladite face ;
- une étape de positionnement d'une paroi, dite d'appui, en matière étanche, souple et extensible, en regard de ladite face de la poche d'empreinteur, et donc en regard de la semelle, de façon à former entre la paroi d'appui et l'empreinte négative un espace dit de formage,
- une étape de formage sous vide, comprenant faire le vide dans l'espace de formage tout en assurant une étanchéité permettant l'établissement du vide, ce par quoi la paroi d'appui vient appuyer la semelle contre l'empreinte négative pour lui donner la forme de cette dernière, et maintenir le vide pendant une durée suffisante pour que la semelle soit conformée ;
- une étape de rupture du vide dans l'espace de formage ; et
- une étape de retrait de la semelle conformée,
le procédé étant caractérisé par le fait que, dans l'étape de mise en place de la semelle, la matière de la face de la poche d'empreinteur est également étanche.

Le retrait de la semelle conformée pourra se faire après déplacement de la paroi d'appui par rapport à la poche d'empreinteur pour exposer l'empreinte négative et la semelle conformée.

Avantageusement, dans l'étape de formage sous vide, le vide peut être maintenu, de préférence de manière constante et uniforme, à un niveau de vide compris entre un vide à 25% et un vide à 100%, de préférence à environ 75%. La pression exercée est alors 2 à 4 fois supérieure à celle exercée par le poids du patient (selon la pointure de la semelle) . On entend par l'expression « vide à 25% », un vide industriel correspondant à -250 mbar.

Avantageusement, dans l'étape de formage sous vide, le vide peut être maintenu pendant une durée indépendante de la capacité du patient à rester debout, correspondant aux exigences du praticien et de la nature de la semelle à conformer. En conséquence de quoi la qualité du formage est améliorée et l'opération de formage se fait en temps masqué, rendant ainsi disponibles le patient et le praticien.

La présente invention a également pour objet un dispositif de formage de semelle destiné à mettre en oeuvre le procédé de formage de semelle tel que défini ci-dessus, le dispositif de formage de semelle comprenant une paroi, dite d'appui, en matière étanche, souple et extensible, destinée à être positionnée en regard de la face de la poche d'empreinteur sur laquelle l'empreinte négative a été réalisée, et donc en regard de la semelle mise en place dans l'empreinte négative, formant l'espace de formage entre la paroi d'appui et l'empreinte négative, le dispositif de formage de semelle comportant à cet effet des moyens de raccordement à une source de vide pour l'établissement du vide dans l'espace de formage et la paroi d'appui étant portée par des moyens de fermeture étanche assurant une étanchéité permettant l'établissement du vide dans l'espace de formage dans l'étape de formage sous vide, et les moyens de fermeture étanche comprennent :
- un bac ayant un fond à partir duquel s'étend une enveloppe latérale se terminant en un rebord périphérique qui délimite une ouverture par laquelle une poche d'empreinteur peut être introduite dans le bac pour être placée sur ledit fond, et
- un cadre qui entoure la paroi d'appui, et auquel cette dernière est fixée, et qui présente un côté inférieur destiné à venir en appui étanche contre le rebord de l'enveloppe latérale de façon à former une chambre délimitée par la paroi d'appui, le fond et l'enveloppe latérale du bac, laquelle chambre inclut l'espace de formage, le cadre et la paroi d'appui formant ensemble un couvercle de fermeture du bac,
le dispositif de formage de semelle étant caractérisé par le fait que les moyens de raccordement comprennent au moins un orifice qui est apte à être mis en communication avec une source de vide et qui débouche sur ladite chambre.

Selon un mode de réalisation préféré, la paroi d'appui est formée par une membrane, de préférence en silicone. En variante, la paroi d'appui pourrait être formée, par exemple, par une paroi d'une vessie gonflable.

Selon un mode de réalisation particulier, les moyens de fermeture étanche comprennent un cadre qui entoure la paroi d'appui, et auquel cette dernière est fixée, et qui présente un côté inférieur destiné à venir en appui étanche contre la partie de la face de la poche d'empreinteur qui entoure l'empreinte négative, le dispositif de formage de semelle se présentant ainsi sous la forme d'une platine de pressage, et les moyens de raccordement comprennent au moins un orifice apte à être mis en communication avec une source de vide.

Dans ce mode de réalisation particulier, le dispositif de formage de semelle peut comprendre des moyens pour presser le cadre contre la poche d'empreinteur, ces moyens comprenant, par exemple, des aimants disposés sur le cadre ou au moins un organe de serrage du cadre et de la poche d'empreinteur l'un contre l'autre, le côté inférieur du cadre étant, de préférence, formé par une couche de matière déformable.

Le fond et l'enveloppe latérale du bac peuvent être rigides. Les parois formant l'enveloppe latérales pourront alors soit être fixes, auquel cas les dimensions du bac seront avantageusement choisies de façon à être légèrement supérieures à celle de l'ensemble poche d'empreinteur pour que les parois latérales de l'enveloppe latérale se trouvent au voisinage immédiat des parois latérales de la poche d'empreinteur, ou en contact en certains endroits avec cette dernière, soit être mobiles par rapport au fond, pour que le praticien puisse les rapprocher des parois latérales de la poche d'empreinteur pour les placer en contact en certains endroits avec cette dernière. Ceci permet à l'enveloppe latérale de s'opposer à une éventuelle déformation de la poche d'empreinteur lors de l'établissement du vide dans ladite chambre, dans le cas où le vide établi dans la poche d'empreinteur n'est pas suffisant pour la rigidifier complètement. On peut ainsi limiter la déformation que subit la poche d'empreinteur, et donc éviter que la forme de l'empreinte négative, notamment dans sa partie inférieure où se situe la semelle, ne soit modifiée par le vide établi dans ladite chambre.

De préférence, le fond et l'enveloppe latérale du bac sont faits d'une matière souple et étanche, de préférence faits de silicone, le rebord périphérique de l'enveloppe latérale étant fixé à un châssis rigide et fixe entourant le bac. Ainsi, lors de l'établissement du vide dans ladite chambre, le fond et l'enveloppe latérale déformables seront plaqués contre le fond et les parois latérales de l'ensemble poche d'empreinteur, de la même manière que la paroi d'appui est plaquée contre la semelle et l'empreinte négative. La paroi d'appui, le fond et les parois latérales du bac épouseront donc l'ensemble poche d'empreinteur, tout autour de ce dernier, conduisant à un équilibre de la force appliquée par la paroi d'appui sur la face supérieure et de la force appliquée par le bac sur les parois latérales et le fond de l'ensemble poche d'empreinteur. Ce système garantit l'intégrité de l'empreinte négative réalisée initialement en évitant tout risque de déformation lié au vide réalisé dans le bac. On a pu constater que l'ensemble poche d'empreinteur conserve sa forme d'origine tout au long de l'étape de formage sous vide, même dans le cas où le vide établi à l'intérieur de la poche d'empreinteur n'est pas suffisant pour le rigidifier de manière optimale.

Comme indiqué ci-dessus, le fond et l'enveloppe latérale du bac peuvent notamment être faits de silicone, sachant que toute autre matière déformable et étanche au vide conviendra.

Au moins un orifice des moyens de raccordement peut être situé dans la région de la paroi d'appui qui est destinée à être en regard de l'empreinte négative dans l'étape de formage sous vide.

De préférence, au moins un orifice est aménagé dans l'enveloppe latérale du bac et le dispositif de formage de semelle comprend en outre au moins un répartiteur de vide, se présentant sous la forme d'un élément d'écartement destiné à s'interposer entre, d'un côté, la paroi d'appui et, d'un autre côté, la région de transition entre la partie de la face de la poche d'empreinteur qui entoure l'empreinte négative et la partie de ladite face qui forme l'empreinte négative. Le ou les répartiteurs de vide assurent la continuité de vide entre la source de vide et la semelle, et permettent ainsi de supprimer le risque que la paroi d'appui vienne se plaquer de manière étanche contre ladite région de transition au début de l'établissement du vide dans ladite chambre, ce qui empêcherait l'air présent dans l'empreinte négative d'être évacué de ladite chambre, et donc empêcherait la paroi d'appui de venir appuyer sur la semelle.

Le ou chaque répartiteur de vide peut être fixé sur la paroi d'appui. La présente invention n'est pas limitée à un nombre particulier ou une disposition particulière du ou des répartiteurs de vide. On pourrait ainsi prévoir quatre éléments d'écartement disposés à 90° les uns des autres, ou un seul élément d'écartement disposé suivant une courbe fermée correspondant à la forme que suit ladite région de transition.

De préférence, le répartiteur de vide est un élément de type fil apte à être posé sur une poche d'empreinteur installée dans le bac, en travers de ladite région de transition.

Avantageusement, le couvercle est relié, de préférence de manière amovible, au bac, le cas échéant au châssis auquel est fixé le rebord périphérique de l'enveloppe latérale du bac, par une liaison pivot d'axe horizontal pour permettre au couvercle de pivoter entre une position de fermeture, dans laquelle le côté inférieur du cadre est en appui contre le rebord de l'enveloppe latérale, et une position d'ouverture, dans laquelle le couvercle a été relevé pour accéder à la poche d'empreinteur en vue de la réalisation de l'empreinte négative initiale ou pour exposer la poche d'empreinteur en vue du retrait de la semelle après qu'elle a été conformée.

La présente invention a également pour objet un système de formage de semelle destiné à mettre en oeuvre le procédé de formage de semelle tel que défini ci-dessus, caractérisé par le fait qu'il comprend au moins un dispositif de formage de semelle tel que défini ci-dessus, une source de vide raccordée au ou à chaque dispositif de formage de semelle par les moyens de raccordement, et des moyens de commande de la source de vide.

En pratique, une fois que le praticien aura rigidifié par le vide la poche d'empreinteur, il lui suffit de positionner l'ensemble poche d'empreinteur par rapport au dispositif de formage de semelle pour que la paroi d'appui soit en regard de la semelle et de l'empreinte négative, puis de commander la source de vide pour mettre en oeuvre l'étape de formage sous vide. Une fois que la semelle est conformée, il commande la source de vide pour casser le vide qui a été établi dans l'espace de formage, puis de déplacer la paroi d'appui et de retirer la semelle conformée.

Dans le mode de réalisation dans lequel le dispositif de formage de semelle se présente sous la forme d'une platine de pressage, positionner l'ensemble poche d'empreinteur consiste simplement à placer le cadre en appui étanche contre la face de la poche d'empreinteur. Dans le mode de réalisation dans lequel le dispositif de formage de semelle comprend un bac et un couvercle, il suffira au praticien de placer l'ensemble poche d'empreinteur dans le bac, puis de fermer ce dernier avec le couvercle.

Selon un mode de réalisation préféré du système, il comprend, par exemple, un ou deux dispositifs de formage de semelle tels que définis dans le mode de réalisation où il comprend un bac et un couvercle, et il comprend en outre des moyens de raccordement supplémentaires s'étendant à travers l'enveloppe latérale, reliés d'un côté à la source de vide et destinés, d'un autre côté, à être mis en communication avec un orifice correspondant d'une poche d'empreinteur lorsque cette dernière est installée sur le fond du bac, de telle sorte que la source de vide est également apte à établir un vide dans la poche d'empreinteur, le système de formage de semelle étant ainsi également apte à permettre la mise en oeuvre de l'étape de réalisation de l'empreinte négative dans une poche d'empreinteur, et constitue ainsi un empreinteur.

Ceci est particulièrement avantageux puisque le praticien n'a plus à manipuler l'ensemble poche d'empreinteur après l'avoir rigidifié par le vide : au lieu de demander au patient de se tenir debout sur la semelle encore tiède, comme cela se fait dans l'état antérieur de la technique, le praticien ferme simplement le bac avec le couvercle, puis actionne la source de vide pour faire le vide dans ladite chambre et ainsi conformer la semelle. Lorsque le système comprend deux dispositifs de formage de semelle, les empreintes négatives pour les deux pieds du patient peuvent être réalisées l'une après l'autre, puis les semelles peuvent être mises en forme sous vide l'une après l'autre ou simultanément. Le patient n'a alors à monter sur les ensembles poches d'empreinteur qu'une seule fois.

Dans ce mode de réalisation préféré, dans le cas où le système comprend deux dispositifs de formage de semelle, la source de vide comprend avantageusement deux pompes à vide ayant chacune quatre têtes, dans lequel :
- les orifices d'aspiration de trois têtes de chaque pompe, dites têtes pour formage, sont reliés aux moyens de raccordement d'un dispositif de formage de semelle respective par un circuit à vide, dit pour formage, dans lequel sont interposés, en partant de la pompe à vide respective, un clapet anti-retour et une électrovanne 3/2, à rappel par ressort dans la position dans laquelle la pompe à vide est à l'échappement, les deux circuits à vide pour formage étant reliés l'un à l'autre en amont des clapets anti-retour ; et
- la quatrième tête de chaque pompe, dite tête pour prise d'empreinte, étant reliée auxdits moyens de raccordement supplémentaires par un circuit à vide, dit pour prise d'empreinte, dans lequel sont interposés, en partant de la pompe à vide respective, une électrovanne 4/2, à rappel par ressort dans la position dans laquelle l'orifice d'aspiration est en communication avec ledit orifice auxiliaire.

Le système pourra comprendre un châssis rigide destiné à être posé au sol et définissant deux compartiments associés chacun à un dispositif de formage de semelle respectif.

Le système peut comprendre en outre, pour chaque dispositif de formage de semelle, un ensemble poche d'empreinteur comprenant une poche d'empreinteur et un bloc de raccordement comprenant un premier moyen de raccordement et un second moyen de raccordement agencés pour se raccorder, de préférence par raccord rapide, respectivement aux moyens de raccordement et aux moyens de raccordement supplémentaires du dispositif de formage de semelle, le premier moyen de raccordement étant agencé pour mettre en communication l'intérieur de la chambre et le circuit à vide pour formage tandis que le second moyen de raccordement est agencé pour mettre en communication l'intérieur de la poche d'empreinteur et le circuit à vide pour prise d'empreinte.

La présente invention a également pour objet un ensemble poche d'empreinteur pour un système tel que défini au paragraphe précédent, l'ensemble poche d'empreinteur comprenant une poche d'empreinteur et un bloc de raccordement, et étant caractérisé par le fait que le bloc de raccordement comprend un premier moyen de raccordement et un second moyen de raccordement agencés pour se raccorder, de préférence par raccord rapide, respectivement aux moyens de raccordement et aux moyens de raccordement supplémentaires du dispositif de formage de semelle, le premier moyen de raccordement étant agencé pour mettre en communication l'intérieur de la chambre et le circuit à vide pour formage tandis que le second moyen de raccordement est agencé pour mettre en communication l'intérieur de la poche d'empreinteur et le circuit à vide pour prise d'empreinte.

Pour mieux illustrer l'objet de la présente invention, on va en décrire ci-après, à titre indicatif et non limitatif, un mode de réalisation préféré avec référence au dessin annexé.

Sur ce dessin :
- la Figure 1 est une vue en perspective d'un système selon un mode de réalisation préféré de la présente invention, sur laquelle on peut voir un ensemble poche d'empreinteur ;
- la Figure 2 est une vue en perspective du châssis du système de la Figure 1 ;
- la Figure 3 est une vue d'un ensemble poche d'empreinteur ;
- la Figure 4 est une vue en perspective du bloc de raccordement de l'ensemble poche d'empreinteur de la Figure 3 ;
- la Figure 5 est une vue en perspective éclatée d'un embout borgne et d'un raccord montés dans le bloc de raccordement ;
- la Figure 6 est une vue en coupe partielle de l'embout borgne et du raccord de la Figure 5, le raccord étant monté dans l'embout borgne ;
- la Figure 7 est une vue en coupe longitudinale de l'ensemble poche d'empreinteur de la Figure 3, prise au voisinage de l'embout ouvert ;
- la Figure 8 est une vue en perspective analogue à la Figure 1, sur laquelle l'ensemble poche d'empreinteur visible sur la Figure 1 a été omis ;
- la Figure 9 est une vue de dessous du système de la Figure 1 ;
- la Figure 10 est une vue en coupe longitudinale du système tel que représenté sur la Figure 6 ;
- la Figure 11 est une vue de détail de la liaison entre le cadre et la paroi d'appui d'un couvercle ;
- la Figure 12 est une vue en coupe longitudinale du système de la Figure 1, le long d'un bord du bac ;
- la Figure 13 est un schéma pneumatique du système de la Figure 1 ; et
- les Figures 14A et 14B sont des vues schématiques montrant le dispositif de formage sous vide et une poche d'empreinteur avant et pendant l'étape de formage sous vide, respectivement.

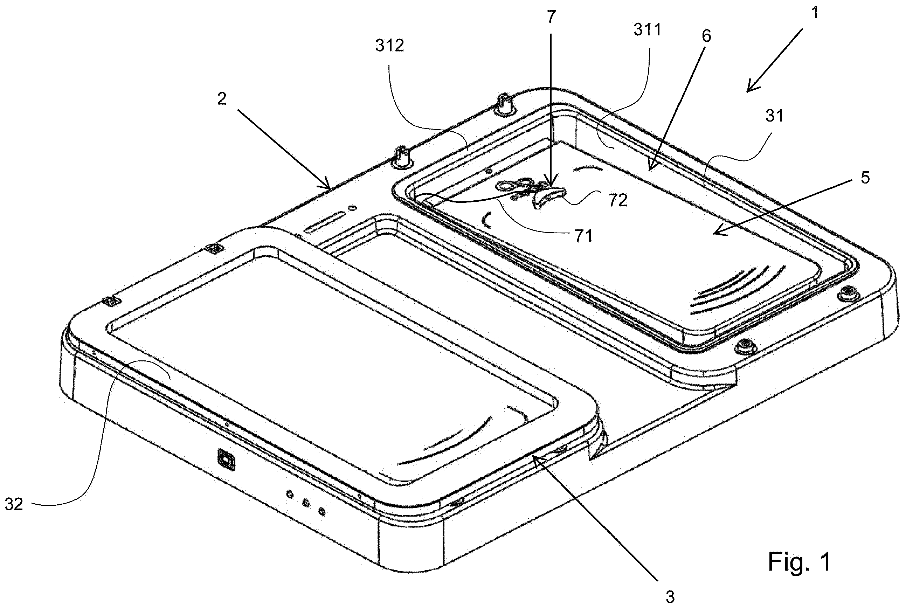

Si l'on se réfère à la Figure 1, on peut voir que l'on y a représenté un système 1 selon un mode de réalisation préféré de la présente invention, qui permet de mettre en oeuvre à la fois l'étape de réalisation des empreintes négatives des deux pieds d'une personne et l'étape de formage des semelles.

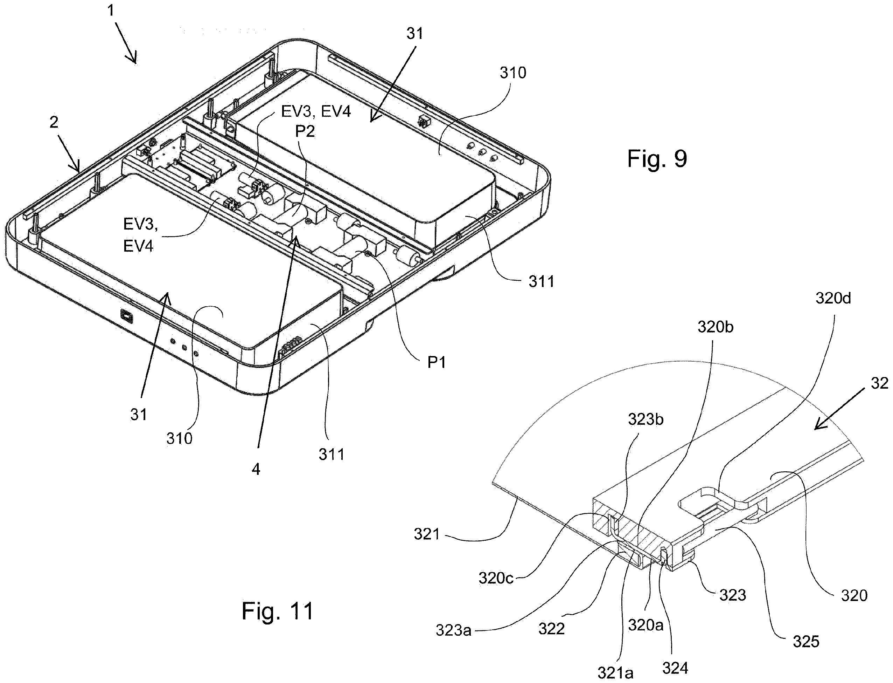

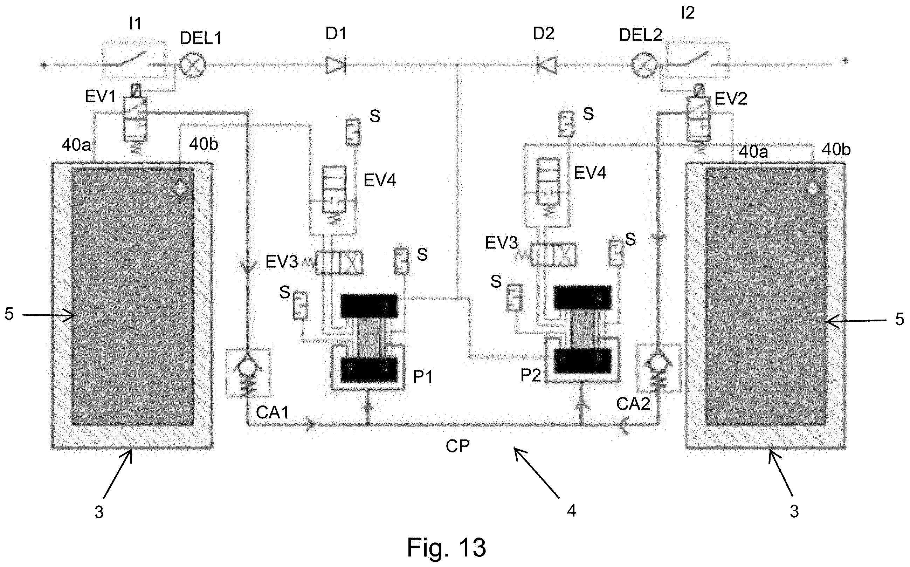

Le système 1 comprend un châssis 2, deux dispositifs de formage de semelle 3 selon un mode de réalisation préféré de la présente invention, une source de vide 4 (visible sur les Figures 9 et 13), des moyens de commande de cette dernière (non représentés), et deux ensembles poches d'empreinteur 5.

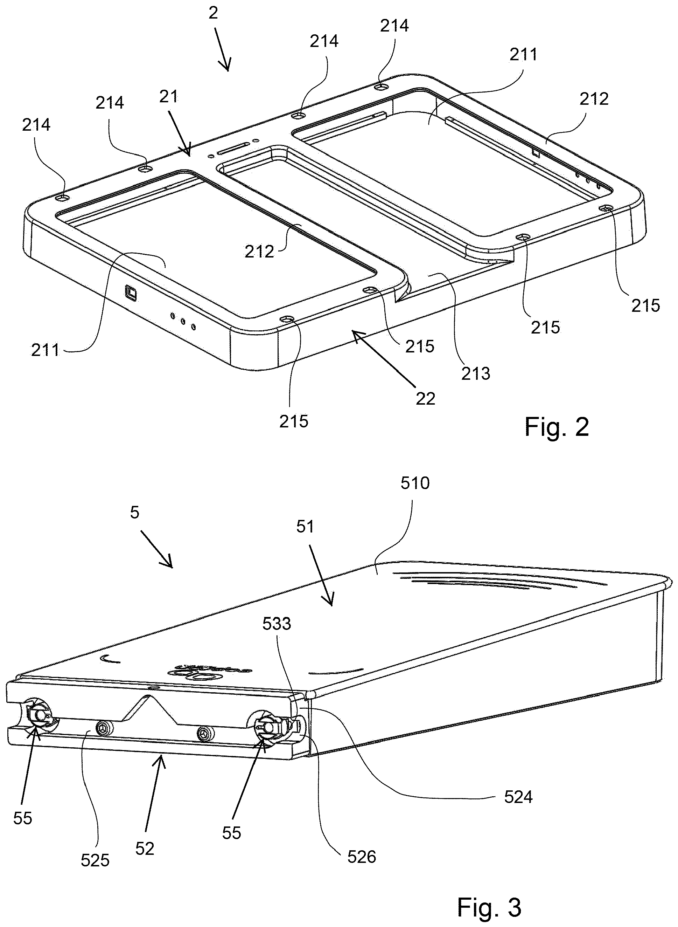

Si l'on se réfère en outre à la Figure 2, on peut voir que le châssis 2, que l'on y a représenté seul, se présente sous la forme d'une pièce creuse du type coque, comprenant une partie supérieure 21 globalement plane et à partir du bord de laquelle s'étend vers le bas une jupe latérale 22. Cette pièce est fermée par une plaque de fermeture (non représentée) fixée au bord libre de la jupe latérale 22.

La partie supérieure 21 comprend deux ouvertures 211 délimitant chacune un compartiment, également désigné par le chiffre de référence 211, destiné à recevoir un dispositif de formage de semelle 3. Les ouvertures 211 sont rectangulaires, de dimensions supérieures à celles des ensembles poches d'empreinteur 5, et donnent sur l'espace intérieur du châssis 2. Les deux ouvertures 211 sont chacune entourées par une région de bord 212 de la partie supérieure 21 et elles sont en outre séparées, dans la direction longitudinale du système 1, par une région centrale 213 de la partie supérieure 21 qui est légèrement renfoncée.

La partie supérieure 21 présente également deux paires de premiers trous traversants 214 le long d'un bord longitudinal du châssis 2 et deux paires de seconds trous traversants 215 le long du bord longitudinal opposé, avec chaque paire au voisinage d'un bord transversal d'une ouverture 211 respective. La fonction des trous traversants 214, 215 sera explicitée ci-après.

Le châssis 2 est réalisé en matière rigide, comme par exemple en matière plastique.

On va maintenant décrire un ensemble poche d'empreinteur 5 selon un mode de réalisation préféré de la présente invention, avec référence aux Figures 3 à 7.

Si l'on se réfère tout d'abord à la Figure 3, on peut voir que l'ensemble poche d'empreinteur 5 comprend une poche d'empreinteur 51 et un bloc de raccordement 52.

La poche d'empreinteur 51 est analogue aux poches d'empreinteur de l'état antérieur de la technique et ne sera donc pas décrite plus en détail ici. Il suffit de souligner que la poche d'empreinteur 51 présente une face supérieure 510 dans laquelle sera réalisée l'empreinte négative, et, dans une paroi d'extrémité axiale 511, deux trous traversants symétriques par rapport au plan longitudinal médian vertical de la poche d'empreinteur 51.

Le bloc de raccordement 52 sert à établir la communication fluidique entre l'espace intérieur de la poche d'empreinteur 51 et la source de vide 4, pour permettre l'établissement d'un vide à l'intérieur de la poche d'empreinteur 51 pour la prise d'une empreinte négative, et la communication fluidique entre l'espace de formage, qui sera décrit plus en détail ci-après, et la source de vide 4, pour l'établissement d'un vide dans l'espace de formage pour le formage sous vide de la semelle.

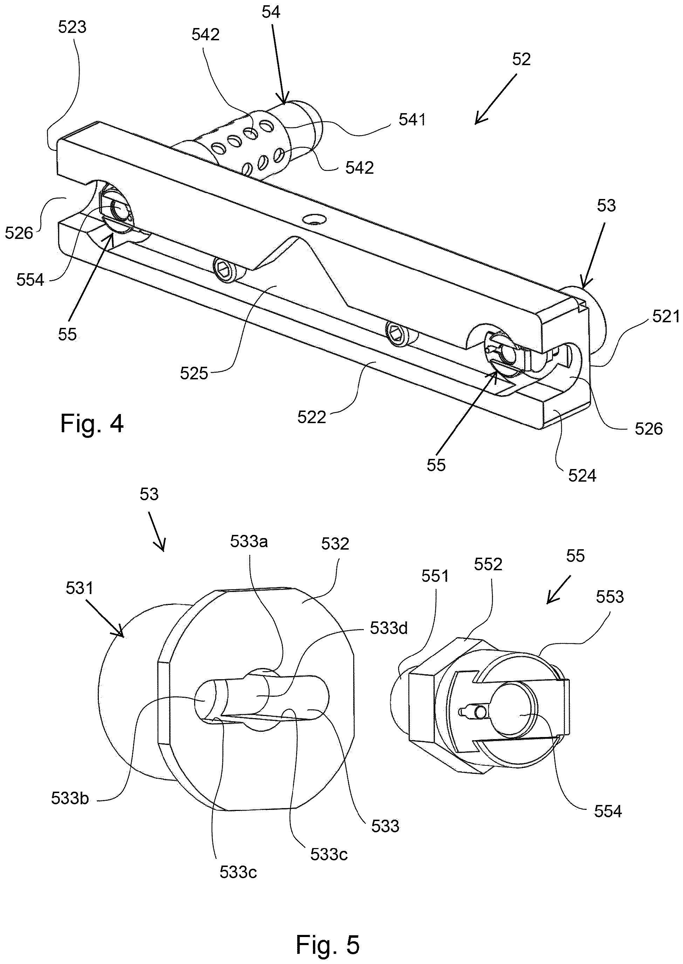

Si l'on se réfère en outre à la Figure 4, on peut voir que le bloc de raccordement 52 est une pièce longitudinale de contour globalement parallélépipédique, présentant ainsi un premier côté longitudinal vertical 521, tourné vers la poche d'empreinteur 51, et un second côté longitudinal vertical 522 opposé audit premier côté longitudinal 521, et deux côtés latéraux verticaux 523, 524. Une rainure 525 s'étend vers l'intérieur à partir du second coté longitudinal 522, d'un côté latéral 523 à l'autre 524, sur lesquels elle débouche en formant un orifice 526. Deux trous traversants (non visibles) s'étendent du fond de la rainure 525 au premier côté 521, l'un au voisinage du côté latéral 523 et l'autre au voisinage du côté latéral 524. Chacun de ces deux trous traversants est aligné respectivement avec l'un des trous traversants que présente la poche d'empreinteur 51.

Un embout borgne 53 est monté dans le trou traversant au voisinage du côté latéral 524, tandis qu'un embout ouvert 54 est monté dans le trou traversant au voisinage du côté latéral 523.

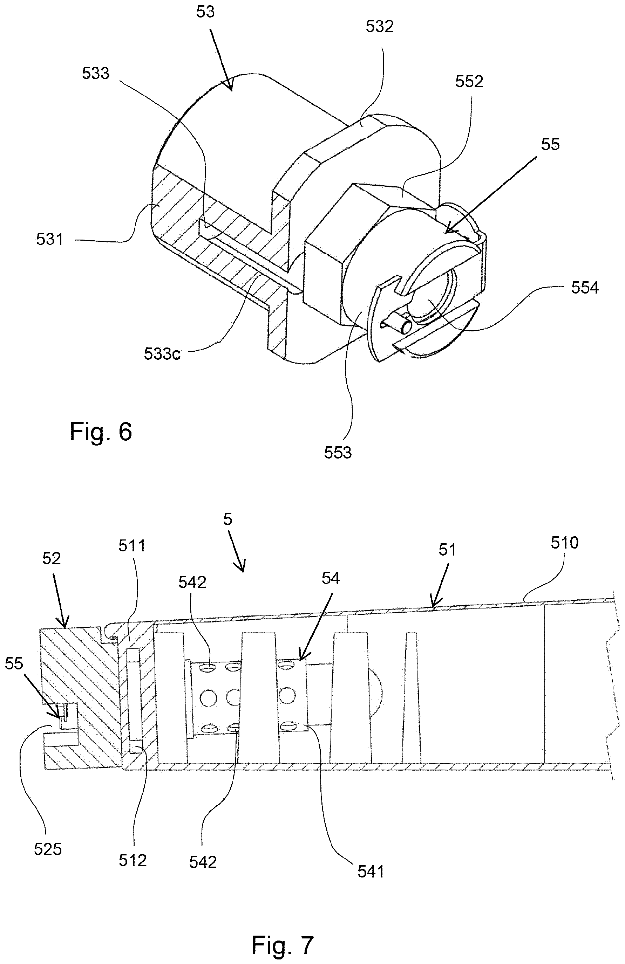

Si l'on se réfère aux Figures 5 et 6, on peut voir que l'embout borgne 53 comprend une partie cylindrique 531 fermée à une extrémité et dont l'autre extrémité présente une bride 532 en contact avec le fond de la rainure 525. Une fente horizontale 533 s'étend vers l'intérieur à partir de la bride 532. La fente 533 comprend une partie centrale arrondie 533a, qui se termine en un trou 533b communiquant avec l'extrémité fermée de la partie cylindrique 531, et deux parties latérales oblongues 533c qui se terminent chacune par une face transversale 533d formant le fond de la fente 533.

On peut également voir sur les Figures 5 et 6 qu'un raccord 55 est monté dans l'embout borgne 53. En particulier, le raccord 55 comprend une partie cylindrique 551 reçue dans la partie centrale arrondie 533a de la fente 533, une collerette 552 venant en appui contre la face externe de la bride 532, et une partie de liaison 553 configurée pour recevoir un embout de raccordement 40a comme cela sera explicité ci-après. A cet effet, un trou traversant 554 s'étend de l'extrémité de la partie cylindrique 551 à la partie de liaison 553.

Si l'on se réfère de manière spécifique à la Figure 6, on peut voir que la longueur de la partie cylindrique 551 du raccord 55 est inférieure à la profondeur de la fente 533, de sorte qu'un intervalle est laissé entre l'extrémité libre de la partie cylindrique 551 et le trou 533b, et donc le fond de la fente 533, ce qui permet de créer un passage latéral vers chacune des deux parties oblongues 533c de la fente 533. De plus, la collerette 552 est dimensionnée de façon à laisser au moins partiellement libre les parties oblongues 533c au niveau de la bride 532, lesquelles communiquent donc avec la rainure 525, et notamment l'orifice 526.

L'embout ouvert 54 comprend une partie cylindrique 541 fermée à une extrémité et dont l'autre extrémité présente une bride (non visible) en contact avec le fond de la rainure 525, de manière analogue à l'embout borgne 53. Un trou axial (non visible) s'étend à partir de la bride jusqu'à l'extrémité fermée et une pluralité de trous radiaux 542 s'étendent à partir de la surface externe de la partie cylindrique 541 et débouchent sur le trou axial.

Un raccord 55 est également monté dans l'embout ouvert 54 et le trou traversant 554 du raccord 55 communique avec le trou axial de l'embout ouvert 54.

Si l'on se réfère maintenant à la Figure 7, on peut voir que la partie cylindrique 541 s'étend à l'intérieur de la poche d'empreinteur 51, et que le bloc de raccordement 52 est fixé à la poche d'empreinteur 51 par liaison, par exemple par collage, des brides 532 des embouts 53, 54 contre la paroi d'extrémité axiale 511 de la poche d'empreinteur, et qu'à cet effet un jonc 512 est noyé dans ladite paroi d'extrémité axiale 511 pour permettre une liaison solide entre le bloc de raccordement 52 et la poche d'empreinteur 51. Le jonc 512 comporte deux orifices qui seront chacun alignés avec l'un respectif des deux trous traversants s'étendant à partir du fond de la rainure 525 et, par conséquent, les deux trous traversants que présentent la poche d'empreinteur 51 et mentionnés ci-dessus sont formés chacun par un orifice prévu dans le jonc 52 et par deux orifices réalisés de part et d'autre du jonc 52 et en regard dudit trou traversant.

On va maintenant décrire le dispositif de formage de semelle 3 selon un mode de réalisation préféré de la présente invention, désigné ci-après par dispositif 3 pour des raisons de concision, avec référence aux Figures 1 et 8 à 12.

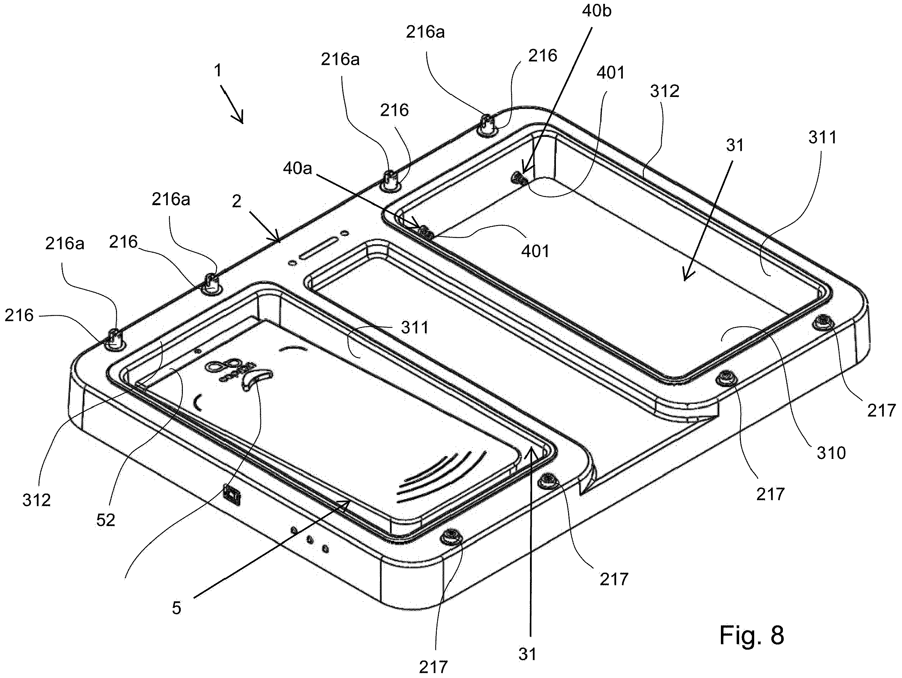

Si l'on se réfère tout d'abord aux Figures 1 et 8 à 10, on peut voir que le dispositif 3 comprend un bac 31 et un couvercle 32. Sur la Figure 1, seul un couvercle 32 a été représenté, et sur la Figure 8 aucun couvercle 32 n'a été représenté et seul un ensemble poche d'empreinteur 5 a été représenté.

Le bac 31 est rectangulaire et comprend un fond 310 et une enveloppe latérale 311 dont l'extrémité libre est repliée vers l'extérieur pour former un rebord 312. Chaque bac 31 est dimensionné pour être introduit à l'intérieur du châssis 2 par l'intermédiaire d'une ouverture 211 respective, et est dimensionné pour être fixé au châssis 2 par fixation, par tout moyen approprié, comme par exemple par collage, du rebord 312 tout autour de la région de bord 212 de l'ouverture 211 respective.

La profondeur du bac 31, en d'autres termes la hauteur de la jupe latérale 311, sera définie pour être au moins légèrement supérieure à la hauteur maximale de l'ensemble poche d'empreinteur 5, de telle sorte que ce dernier peut être installé dans le bac 31, en reposant sur le fond 310 du bac 31, sans que la face supérieure 510 de la poche d'empreinteur 51 ne se situe au-dessus du rebord 312.

L'enveloppe latérale 311 comporte dans une paroi parallèle à la direction longitudinale du système 1 deux trous traversants positionnés de façon à être en regard des raccords 55 lorsque l'ensemble poche d'empreinteur 5 est installé dans le bac 31. Chacun de ces deux trous traversants est traversé par un embout de raccordement 40a, 40b, dont une partie 401 fait saillie dans le bac 31 et dont une autre partie 402 se situe hors du bac 31 et est reliée à la source de vide 4.

Le bac 31 est réalisé dans une matière souple et étanche, de préférence en silicone.

Si l'on se réfère maintenant également à la Figure 11, on peut voir que le couvercle 32 comprend un cadre 320 et une membrane 321 s'étendant en travers de celui-ci.

Le cadre 320 est rigide et rectangulaire, de dimensions au moins légèrement supérieures à celles du bac 31.

La membrane 321 forme la paroi d'appui selon la présente invention, et est ainsi en une matière souple, étanche et extensible, de préférence en silicone.

La membrane 321 peut être fixée par tout moyen approprié au cadre 320. Par exemple, comme illustré sur la Figure 11, la région de bord 321a de la membrane 321 peut être repliée vers l'intérieur autour d'un jonc annulaire 322, de forme rectangulaire, pour être prise en sandwich entre le jonc annulaire 322 et un côté inférieur 320a du cadre 320. Pour obtenir une liaison solide entre la membrane 321 et le cadre 320, le jonc annulaire 322 est vissé au cadre 320 par une pluralité de vis réparties le long du cadre 320 et qui se vissent dans le jonc annulaire 322 en passant par des trous de vis respectifs prévus dans le cadre 320 et en traversant la région de bord 321a de la membrane 321.

Dans l'exemple illustré, ce côté inférieur 320a est formé par une pièce en L rapportée 323, dont la branche longue 323a est appliquée contre une face en retrait 320b du cadre 320 et y est fixée par des vis 324, côté extérieur du cadre 320, et dont la branche courte 323b s'étend à l'intérieur d'une rainure 320c prévue de manière correspondante dans le cadre 320, côté intérieur du cadre 320.

Toujours avec référence avec référence à la Figure 11, on peut y voir une rainure verticale 320d s'étendant vers l'intérieur à partir de la face verticale côté extérieur du cadre 320. En réalité, il est prévu deux de ces rainures verticales 320d, en travers de chacune desquelles s'étend un pivot 325. Chacun de ces pivots 325 est destiné à être introduit dans une fente verticale 216a que présente un plot 216 faisant saillie hors de l'un respectif des trous traversants 214 du châssis 2. Les pivots 325 et les fentes verticales 216a sont dimensionnés pour que les pivots 325 puissent tourner sur eux-mêmes après avoir été introduits dans les fentes verticales 216a. Ainsi, chaque couvercle 32 peut être monté à la fois de manière amovible sur le châssis 2 et de manière pivotante autour d'un axe de pivot horizontal parallèle à la direction longitudinale du châssis 2.

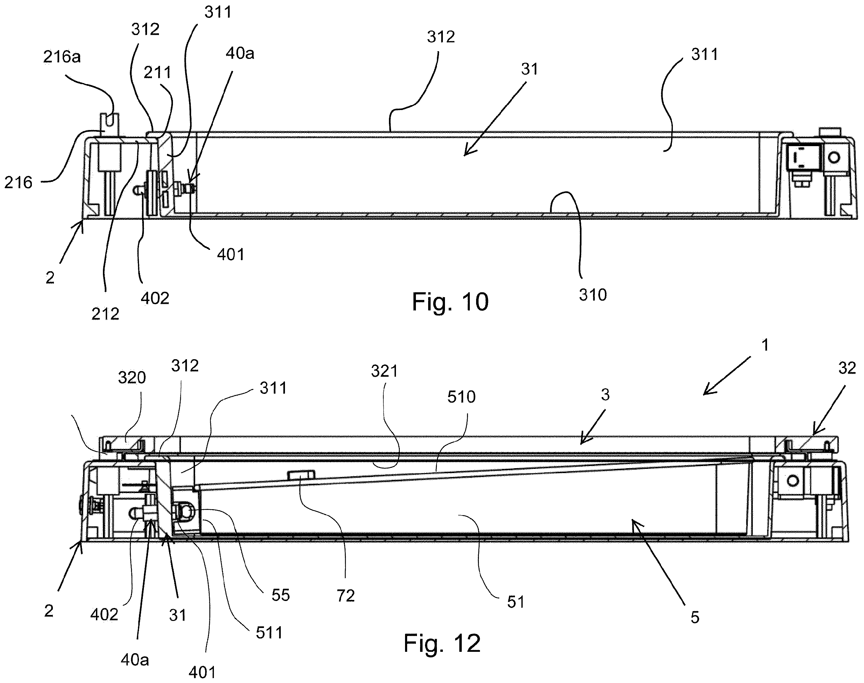

Chaque couvercle 32 peut ainsi être pivoté entre une position d'ouverture, dans laquelle il est relevé pour donner accès au bac 31, et une position de fermeture dans laquelle il ferme le bac 31, avec le cadre 320 en appui contre le bord 312 du bac 31, comme on peut le voir sur la Figure 12. Dans la position de fermeture, le couvercle 32 repose à son extrémité libre sur deux plots d'appui 217 faisant saillie hors des trous traversants 215 du châssis 2.

On souligne ici que cet appui du cadre 320 peut se faire directement, à savoir que le côté inférieur 320a du cadre 320 vient directement en contact avec le bord 312 du bac 31, ou se faire indirectement, à savoir que des parties sont interposées entre ledit côté inférieur 320a et ledit bord 312. Par exemple, dans l'exemple illustré sur les Figures 11 et 12, une partie de la membrane 321 est prise en sandwich entre le cadre 320 et le bord 312 du bac 31, ici la partie qui se situe au voisinage du jonc annulaire 322, côté intérieur par rapport à ce dernier. Bien entendu, on pourrait imaginer que le cadre 320 ou la membrane 321 vienne directement en contact sur la région de bord 212 du châssis 2 dans lequel le bac 31 est fixé d'une autre manière au châssis 2, par exemple avec le bord 312 du bac 31 sous la partie supérieure 21 du châssis 2.

On souligne également que cet appui est étanche. Dans le mode de réalisation représenté, cette étanchéité peut être assurée par le seul poids du couvercle 32, qui est suffisamment élevé, mais l'on pourrait prévoir des moyens pour serrer le couvercle 32 contre le châssis 2, par exemple à l'aide d'aimants, de sangles de serrage, de brides, etc.

Comme on peut le voir sur la Figure 12, dans la position de fermeture, le bac 31 et le couvercle 32 définissent ensemble une chambre 6 dans laquelle est installé l'ensemble poche empreinteur 5. La chambre 6 ne communique avec l'extérieur que par l'intermédiaire d'un passage formé par l'orifice 526 dans le côté latéral 524 du bloc de raccordement 52, la rainure 525 du raccord 55, la fente horizontale 533 de l'embout borgne 53, puis le trou traversant 554 du raccord 55 et l'embout de raccordement 40a. On peut notamment voir sur la Figure 12 que la partie 401 de l'embout de raccordement 40a est reçue dans le trou traversant 554 du raccord 55 lorsque l'ensemble poche d'empreinteur 5 est correctement installé dans le bac 51. Dans cette position, le raccord 55 monté dans l'embout ouvert 54 reçoit de manière analogue à l'embout de raccordement supplémentaire 40b.

Chaque embout de raccordement 40a, 40b est relié à la source de vide 4 que l'on va maintenant décrire avec référence aux Figures 9 et 13.

La source de vide 4 comprend deux pompes à vide P1, P2 ayant chacune quatre têtes de pompe. Chaque pompe à vide P1, P2 est reliée à un embout de raccordement 40a respectif par un circuit à vide pour formage et à un embout de raccordement supplémentaire 40b respectif par un circuit à vide pour prise d'empreinte.

Chaque circuit à vide pour formage comprend plusieurs conduits reliant les orifices d'aspiration de trois têtes de la pompe à vide P1, P2 respective à un conduit principal CP relié à une extrémité à un embout de raccordement 40a et l'autre extrémité à l'autre embout de raccordement 40a. En amont (dans le sens d'aspiration de l'air) de chaque pompe à vide P1, P2 est prévu un clapet anti-retour CA1, CA2, et en amont de chaque clapet anti-retour CA1, CA2 es prévu une électrovanne 3/2, à rappel par ressort dans la position dans laquelle la pompe à vide P1, P2 est à l'échappement. Cette électrovanne est désignée par le signe de référence EV1, EV2.

L'orifice d'échappement de chacune de ces trois têtes de chaque pompe à vide P1, P2, est relié à l'air libre par une conduite d'échappement dans laquelle est placé un silencieux d'échappement S.

A chaque électrovanne EV1, EV2 est associé un interrupteur marche/arrêt I1, I2 en aval duquel est montée une diode électroluminescente DEL1, DEL2 et une diode D1, D2 laissant passant le courant provenant de l'interrupteur I1, I2 respectif, lequel courant sert à actionner les pompes à vide P1, P2.

Ainsi, lorsqu'un interrupteur I1, I2 est fermé, la diode électroluminescente DEL1, DEL2 associée s'allume, les pompes à vide P1, P2 sont amenées à fonctionner et l'électrovanne EV1, EV2 se place dans la position dans laquelle la conduite principale CP communique avec l'embout de raccordement 40a. Comme indiqué ci-dessus, l'embout de raccordement 40a communique avec la chambre 6, de sorte que les pompes à vide P1, P2 font le vide dans la chambre 6. L'utilisateur est informé de la mise sous vide du fait que la diode DEL1, DEL2 s'allume.

On souligne ici que le montage illustré sur la Figure 13 permet une commande indépendante pour chacun des dispositifs de formage de semelle 3 : le courant provenant d'un interrupteur I1 est bloqué par la diode D2 associée à l'interrupteur I2, de sorte que si ce dernier reste en position ouvert, l'électrovanne EV2 associée reste dans sa position rappelée par le ressort et l'embout de raccordement 40a correspondant n'est pas mis en communication avec les pompes à vide P1, P2.

Ce montage présente également l'avantage de faire établir le vide dans une chambre 6 respective par les deux pompes à vide P1, P2, ce qui permet d'atteindre dans la chambre 6 une valeur de vide suffisante pour que l'appui de la membrane 321 contre la semelle conforme cette dernière, et, dans le cas d'une semelle thermoformable, d'une manière suffisamment rapide pour que cette valeur de vide soit atteinte alors que la semelle n'a pas encore refroidie et peut donc être déformée par la membrane 321.

Ce montage permet également, si on le souhaite, d'établir le vide simultanément dans les deux chambres 6 si l'on souhaite mettre en forme deux semelles simultanément.

Chaque circuit à vide pour prise d'empreinte comprend un premier conduit reliant l'orifice d'aspiration de la quatrième tête de la pompe à vide P1, P2 respective à un embout de raccordement supplémentaire 40b et un second conduit reliant à l'air libre l'orifice d'échappement. Une électrovanne 4/2, à rappel par ressort dans la position dans laquelle l'orifice d'aspiration est en communication avec l'embout de raccordement supplémentaire 40b et l'orifice d'échappement est à l'échappement, est prévue en amont de l'orifice d'aspiration/en aval de l'orifice d'échappement. Cette électrovanne est désignée par le signe de référence EV3. Un silencieux est placé sur la ligne d'échappement, en aval de l'électrovanne EV3. Une électrovanne 2/2, à rappel par ressort dans la position fermée, désignée par le signe de référence EV4, est montée en parallèle de l'électrovanne EV3 de façon à relier, dans la position ouverte, le conduit reliant l'électrovanne EV3 à l'embout de raccordement supplémentaire 40b et la ligne d'échappement menant à l'air libre, et dans laquelle est prévu, en aval, un silencieux S.

Ainsi, lorsque les pompes à vide P1, P2 sont actionnées, elles pourront établir un vide dans la poche d'empreinteur 51. Il est également possible de casser le vide à l'intérieur de la poche d'empreinteur 51 alors qu'elle est encore installée dans le bac 31, en commandant les électrovannes EV3 et EV4 pour les placer dans leur seconde position, dans laquelle l'orifice d'aspiration et l'orifice d'échappement de la quatrième tête sont reliés respectivement à la ligne d'échappement et à la conduite menant à l'embout de raccordement supplémentaire 40b, laquelle conduite est par ailleurs mise à l'échappement par l'électrovanne EV4.

Par conséquent, le système 1 selon le mode de réalisation préféré décrit ci-dessus permet au praticien de réaliser l'ensemble de la fabrication d'une ou de deux semelles sans manipuler les ensembles poches d'empreinteur 5.

Tout d'abord, le praticien place chaque ensemble poche d'empreinteur 5 dans un bac 31 respectif, si elles ne s'y trouvent déjà pas, où les ensembles poches d'empreinteur 5 se raccordent automatiquement à la source de vide 4 via les circuits à vide pour prise d'empreinte et les circuits à vide pour formage.

De manière classique, le patient vient placer un pied sur une poche d'empreinteur 51, l'autre pied reposant sur la région centrale 213 du châssis 2. Le praticien modèle la poche d'empreinteur 51 pour obtenir l'empreinte négative souhaitée, puis place une semelle thermoformable, chauffée au préalable, dans l'empreinte négative. Le praticien commande ensuite la source de vide 4 pour faire le vide dans la poche d'empreinteur 51 et ainsi figer l'empreinte négative.

Le praticien place ensuite un répartiteur de vide 7, qui se présente ici sous la forme d'un élément d'écartement de type fil, comme par exemple une chaîne 71 représentée schématiquement uniquement sur la Figure 1, dont une extrémité est reliée à une pièce en demi-lune 72 (Figures 1 et 12), la chaîne 71 passant à travers un trou prévu à cet effet dans le châssis 2. Ainsi, le praticien peut saisir la chaîne 71 par la pièce en demi-lune 72, la tirer, puis la placer au voisinage de l'extrémité avant de la semelle de sorte que la chaîne 71 s'étende en travers de la région de transition entre l'empreinte négative et la partie de la face supérieure 510 de la poche d'empreinteur 51 entourant l'empreinte négative. On assure ainsi la continuité du vide entre la chambre 6 et la source de vide 4 tout au long de l'étape de formage sous vide.

Le praticien ferme ensuite le bac 31 en faisant pivoter le couvercle 32 vers sa position de fermeture, puis il actionne la source de vide 4 pour établir le vide dans la chambre 6 et ainsi former sous vide la semelle.

Le vide est maintenu pendant une durée suffisante pour que la semelle soit conformée et refroidie.

Une fois ce délai écoulé, le praticien commande la source de vide 4 pour mettre la chambre 6 à l'échappement et ainsi rompre le vide établi dans la chambre 6, puis il peut relever le couvercle 32 et retirer de l'empreinte négative la semelle conformée et refroidie. Le praticien met également l'ensemble poche d'empreinteur 5 à l'échappement en commandant la source de vide 4, soit avant soit après avoir retiré la semelle de l'empreinte négative.

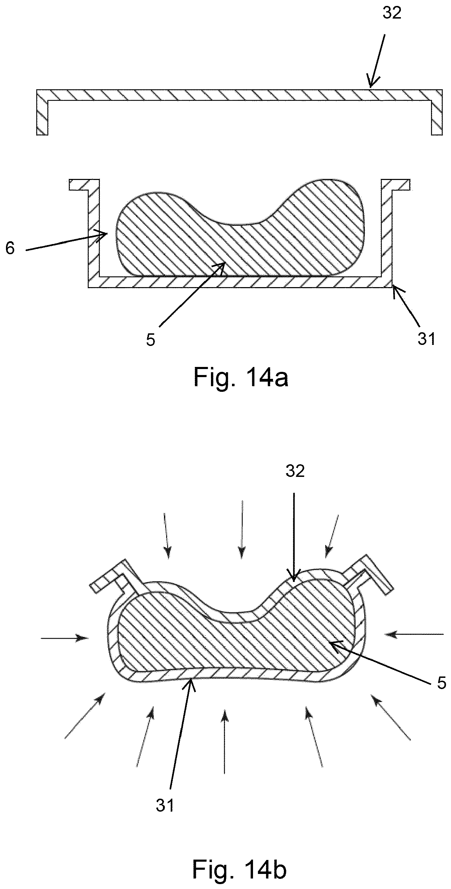

On souligne ici que la semelle obtenue est conformée correctement et aura épousé parfaitement la forme de l'empreinte négative du fait de la déformation de la membrane 321 et du fond 310 et de l'enveloppe latérale 312 du bac 31 lors de l'étape de formage sous vide, comme cela est illustré sur les Figures 14A et 14B.

Sur la Figure 14A, on peut voir qu'avant l'étape de formage sous vide, la membrane 321 et l'enveloppe latérale 312 du bac 31 sont à distance de la poche d'empreinteur 51. Ce schéma correspond à ce que l'on peut voir sur la Figure 12.

Sur la Figure 14B, on peut voir que pendant l'étape de formage sous vide, l'établissement du vide dans la chambre 6 a déformé la membrane 321, le fond 310 et l'enveloppe latérale 311 en les plaquant contre la poche d'empreinteur 5. On a pu constater par des essais que la forme de la poche d'empreinteur 5, et donc de l'empreinte négative, reste inchangée tout au long de l'étape de formage sous vide, ce qui est dû au fait que les forces appliquées sur la poche d'empreinteur 5 se répartissent tout autour de cette dernière et une fois le vide effectué la pression s'équilibre.

L'utilisation d'un bac 31 en une matière souple et étanche, permet donc de garantir le maintien de la forme de l'empreinte négative même lorsque la poche d'empreinteur 5 est susceptible de se déformer car le vide établi à l'intérieur de celle-ci pour figer l'empreinte négative ne serait pas suffisant pour interdire une éventuelle déformation due à la pression plus importante appliquée sur la poche d'empreinteur 5 lors de l'établissement du vide dans la chambre 6.

Il est bien entendu que le mode de réalisation préféré qui vient d'être décrit a été donné à titre indicatif et non limitatif et que des modifications peuvent être apportées sans que l'on s'écarte pour autant du cadre de la présente invention.

## Revendications

1. Procédé de formage d'une semelle dans une empreinte négative, comprenant les étapes successives suivantes :
- une étape de mise en place d'une semelle sur une face (510) d'une poche d'empreinteur (5), ladite face (510) étant en matière souple, extensible, et la poche d'empreinteur (5) étant remplie d'une charge qui autorise une déformation de ladite face (510) ;
- une étape de positionnement d'une paroi (321), dite d'appui, en matière étanche, souple et extensible, en regard de ladite face (510) de la poche d'empreinteur (5), et donc en regard de la semelle, de façon à former entre la paroi d'appui (321) et l'empreinte négative un espace dit de formage (6),
- une étape de formage sous vide, comprenant faire le vide dans l'espace de formage (6) tout en assurant une étanchéité permettant l'établissement du vide, ce par quoi la paroi d'appui (321) vient appuyer la semelle contre l'empreinte négative pour lui donner la forme de cette dernière, et maintenir le vide pendant une durée suffisante pour que la semelle soit conformée ;
- une étape de rupture du vide dans l'espace de formage (6) ; et
- une étape de retrait de la semelle conformée,
le procédé étant **caractérisé par le fait que**, dans l'étape de mise en place de la semelle, la matière de la face (510) de la poche d'empreinteur (5) est également étanche.

2. Procédé de formage de semelle selon la revendication 1, **caractérisé par le fait que**, dans l'étape de formage sous vide, le vide est maintenu, de préférence de manière constante et uniforme, à un niveau de vide compris entre un vide à 25% et un vide à 100%, de préférence à environ 75%.

3. Dispositif de formage de semelle (3) destiné à mettre en oeuvre le procédé de formage de semelle tel que défini à l'une quelconque des revendications 1 et 2, le dispositif de formage de semelle (3) comprenant une paroi (321), dite d'appui, en matière étanche, souple et extensible, destinée à être positionnée en regard de la face (510) de la poche d'empreinteur (51) sur laquelle l'empreinte négative a été réalisée, et donc en regard de la semelle mise en place dans l'empreinte négative, formant l'espace de formage (6) entre la paroi d'appui (321) et l'empreinte négative, le dispositif de formage de semelle (3) comportant à cet effet des moyens de raccordement (40a) à une source de vide (4) pour l'établissement du vide dans l'espace de formage (6) et la paroi d'appui (321) étant portée par des moyens de fermeture étanche (31, 32) assurant une étanchéité permettant l'établissement du vide dans l'espace de formage (6) dans l'étape de formage sous vide, et :
- les moyens de fermeture étanche (31, 32) comprennent :
- un bac (31) ayant un fond (310) à partir duquel s'étend une enveloppe latérale (311) se terminant en un rebord périphérique (312) qui délimite une ouverture par laquelle une poche d'empreinteur (51) peut être introduite dans le bac (31) pour être placée sur ledit fond (310), et
- un cadre (320) qui entoure la paroi d'appui (321), et auquel cette dernière est fixée, et qui présente un côté inférieur (320a) destiné à venir en appui étanche contre le rebord (312) de l'enveloppe latérale (311) de façon à former une chambre (6) délimitée par la paroi d'appui (321), le fond (310) et l'enveloppe latérale (311) du bac (3), laquelle chambre (6) inclut l'espace de formage, le cadre (320) et la paroi d'appui (321) formant ensemble un couvercle (32) de fermeture du bac (31),
le dispositif de formage de semelle (3) étant **caractérisé par le fait que** les moyens de raccordement (40a) comprennent au moins un orifice qui est apte à être mis en communication avec une source de vide (4) et qui débouche sur ladite chambre (6).

4. Dispositif de formage de semelle (3) selon la revendication 3, **caractérisé par le fait que** la paroi d'appui est formée par une membrane (321), de préférence en silicone.

5. Dispositif de formage de semelle (3) selon l'une quelconque des revendications 3 et 4, **caractérisé par le fait que** le fond (310) et l'enveloppe latérale (311) du bac (31) sont faits d'une matière souple et étanche, de préférence faits de silicone, le rebord périphérique (312) de l'enveloppe latérale (311) étant fixé à un châssis (2) rigide et fixe entourant le bac (31).

6. Dispositif de formage de semelle selon l'une quelconque des revendications 3 à 5, **caractérisé par le fait qu'**au moins un orifice des moyens de raccordement est situé dans la région de la paroi d'appui qui est destinée à être en regard de l'empreinte négative dans l'étape de formage sous vide.

7. Dispositif de formage de semelle (3) selon l'une quelconque des revendications 3 à 5, **caractérisé par le fait qu'**au moins un orifice est aménagé dans l'enveloppe latérale (311) du bac (31) et que le dispositif de formage de semelle (3) comprend en outre au moins un répartiteur de vide (7), se présentant sous la forme d'un élément d'écartement (71) destiné à s'interposer entre, d'un côté, la paroi d'appui (321) et, d'un autre côté, la région de transition entre la partie de la face (510) de la poche d'empreinteur (51) qui entoure l'empreinte négative et la partie de ladite face (510) qui forme l'empreinte négative.

8. Dispositif de formage de semelle (3) selon la revendication 7, **caractérisé par le fait que** le répartiteur de vide (7) est un élément de type fil (71) apte à être posé sur une poche d'empreinteur (51) installée dans le bac (31), en travers de ladite région de transition.

9. Dispositif de formage de semelle (3) selon l'une quelconque des revendications 3 à 8, **caractérisé par le fait que** le couvercle (32) est relié, de préférence de manière amovible, au bac (31), le cas échéant au châssis (2) auquel est fixé le rebord périphérique (312) de l'enveloppe latérale (311) du bac (31), par une liaison pivot d'axe horizontal pour permettre au couvercle (32) de pivoter entre une position de fermeture, dans laquelle le côté inférieur (320a) du cadre (320) est en appui contre le rebord (312) de l'enveloppe latérale (311), et une position d'ouverture, dans laquelle le couvercle (32) a été relevé pour accéder à la poche d'empreinteur (51) en vue de la réalisation de l'empreinte négative initiale ou pour exposer la poche d'empreinteur (51) en vue du retrait de la semelle après qu'elle a été conformée.

10. Système de formage de semelle (1) destiné à mettre en oeuvre le procédé de formage de semelle tel que défini à l'une quelconque des revendications 1 et 2, **caractérisé par le fait qu'**il comprend au moins un dispositif de formage de semelle (3) tel que défini à l'une des revendication 3 à 9, une source de vide (4) raccordée au ou à chaque dispositif de formage de semelle (3) par les moyens de raccordement (40a), et des moyens de commande de la source de vide (4).

11. Système (1) selon la revendication 10, comprenant un ou deux dispositifs de formage de semelle (3) tels que définis à l'une quelconque des revendications 3 à 9, **caractérisé par le fait qu'**il comprend des moyens de raccordement supplémentaires (40b) s'étendant à travers l'enveloppe latérale (311), reliés d'un côté à la source de vide (4) et destinés, d'un autre côté, à être mis en communication avec un orifice correspondant d'une poche d'empreinteur (51) lorsque cette dernière est installée sur le fond (310) du bac (31), de telle sorte que la source de vide (4) est également apte à faire un vide dans la poche d'empreinteur (51), le système de formage de semelle (1) étant ainsi également apte à permettre la mise en oeuvre de l'étape de réalisation de l'empreinte négative dans une poche d'empreinteur (51), et constitue ainsi un empreinteur.

12. Système selon la revendication 11, comprenant deux dispositifs de formage de semelle (3), **caractérisé par le fait que** la source de vide (4) comprend deux pompes à vide (P1, P2) ayant chacune quatre têtes, dans lequel :
- les orifices d'aspiration de trois têtes de chaque pompe, dites têtes pour formage, sont reliés aux moyens de raccordement (40a) d'un dispositif de formage de semelle (3) respective par un circuit à vide, dit pour formage, dans lequel sont interposés, en partant de la pompe à vide respective (P1, P2), un clapet anti-retour (CA1, CA2) et une électrovanne (EV1, EV2) 3/2, à rappel par ressort dans la position dans laquelle la pompe à vide (P1, P2) est à l'échappement, les deux circuits à vide pour formage étant reliés l'un à l'autre en amont des clapets anti-retour (CA1, CA2) ; et
- la quatrième tête de chaque pompe, dite tête pour prise d'empreinte, étant reliée auxdits moyens de raccordement supplémentaires par un circuit à vide, dit pour prise d'empreinte, dans lequel sont interposés, en partant de la pompe à vide respective (P1, P2), une électrovanne (EV3) 4/2, à rappel par ressort dans la position dans laquelle l'orifice d'aspiration est en communication avec ledit orifice auxiliaire.

13. Système (1) selon la revendication 12, **caractérisé par le fait qu'**il comprend un châssis rigide (2) destiné à être posé au sol et définissant deux compartiments (211) associés chacun à un dispositif de formage de semelle (3) respectif.

14. Système (1) selon l'une quelconque des revendications 12 et 13, **caractérisé par le fait qu'**il comprend en outre, pour chaque dispositif de formage de semelle (3), un ensemble poche d'empreinteur (5) comprenant une poche d'empreinteur (51) et un bloc de raccordement (52) comprenant un premier moyen de raccordement(55, 53, 525, 526) et un second moyen de raccordement (55, 54) agencés pour se raccorder, de préférence par raccord rapide, respectivement aux moyens de raccordement (40a) et aux moyens de raccordement supplémentaires (40b) du dispositif de formage de semelle (3), le premier moyen de raccordement (55, 53, 525, 526) étant agencé pour mettre en communication l'intérieur de la chambre (6) et le circuit à vide pour formage tandis que le second moyen de raccordement (55, 54) est agencé pour mettre en communication l'intérieur de la poche d'empreinteur (51) et le circuit à vide pour prise d'empreinte.

15. Ensemble poche d'empreinteur (5) pour un système (1) tel que défini à la revendication 14, l'ensemble poche d'empreinteur (5) comprenant une poche d'empreinteur (51) et un bloc de raccordement (52), **caractérisé par le fait que** le bloc de raccordement (52) comprend un premier moyen de raccordement (55, 53, 525, 526) et un second moyen de raccordement (55, 54) agencés pour se raccorder, de préférence par raccord rapide, respectivement aux moyens de raccordement (40a) et aux moyens de raccordement supplémentaires (40b) du dispositif de formage de semelle (3), le premier moyen de raccordement (55, 53, 525, 526) étant agencé pour mettre en communication l'intérieur de la chambre (6) et le circuit à vide pour formage tandis que le second moyen de raccordement (55, 54) est agencé pour mettre en communication l'intérieur de la poche d'empreinteur (51) et le circuit à vide pour prise d'empreinte.
